(19) Europäisches Patentamt
European Patent Office
Office européen des brevets

(11) EP 2 796 441 A2

(12) # DEMANDE DE BREVET EUROPEEN

(43) Date de publication:
**29.10.2014 Bulletin 2014/44**

(51) Int Cl.:
***C07C 51/353*** *(2006.01)* ***C07C 59/52*** *(2006.01)*

(21) Numéro de dépôt: **14177944.7**

(22) Date de dépôt: **22.07.2014**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **26.05.2014 FR 1401206**

(71) Demandeur: **Rhodia Opérations**
**75009 Paris (FR)**

(72) Inventeurs:
- **Vidal, Jean-Paul**
  **69390 Vourles (FR)**
- **Pitiot, Pascal**
  **69008 Lyon (FR)**

(74) Mandataire: **Menville, Laure**
**Rhodia Opérations**
**IAM**
**85, avenue des frères Perret**
**69192 Saint-Fons (FR)**

(54) **Procédé de préparation de composés p-hydroxymandéliques en réacteurs agités**

(57) Le procédé permet de préparer un composé p-hydroxymandélique comprenant au moins une étape de condensation d'au moins un composé aromatique porteur d'au moins un groupe hydroxyle et dont la position en para est libre, avec l'acide glyoxylique, la réaction de condensation étant conduite dans au moins un réacteur muni d'au moins un moyen de mélange, la puissance spécifique de mélange étant comprise entre 0,1 kW/$m^3$ et 15 kW/$m^3$. En outre, l'invention a aussi pour objet un procédé de préparation d'un aldéhyde 4-hydroxyaromatique par oxydation de ce composé p-hydroxymandélique.

EP 2 796 441 A2

## Description

DOMAINE TECHNIQUE DE L'INVENTION

La présente invention concerne la préparation de composés p-hydroxymandéliques, c'est-à-dire de composés aromatiques porteur d'au moins un groupe -CHOH-COOH en position para d'un groupe hydroxyle. Plus spécifiquement, cette invention concerne un procédé de préparation d'un composé p-hydroxymandélique par condensation d'un composé aromatique porteur d'au moins un groupe hydroxyle et dont la position en para est libre, avec l'acide glyoxylique.

De préférence, le composé p-hydroxymandélique préparé est l'acide 4-hydroxy-3-méthoxy-mandélique ou l'acide 4-hydroxy-3-éthoxy-mandélique.

ETAT DE LA TECHNIQUE

La vanilline peut être obtenue à partir de sources naturelles telles que la lignine ou l'acide férulique, mais une part importante de la vanilline est produite par voie chimique.

De nombreuses méthodes de préparation diverses et variées sont décrites dans la littérature (KIRK-OTHMER - Encyclopedia of Chemical Technology 24, p 812-825, 4ème édition (1997)).

Une voie d'accès classique à la vanilline implique une réaction de condensation d'acide glyoxylique sur le gaïacol en milieu basique pour obtenir l'acide 4-hydroxy-3-méthoxymandélique. Ce produit est ensuite oxydé pour conduire à la vanilline.

Une des difficultés techniques de cette réaction réside dans le fait que le rendement de la condensation est limité par son manque de sélectivité. Outre l'acide p-hydroxymandélique, cette réaction conduit également à l'acide o-hydroxymandélique, un produit issu d'une réaction parasite, ainsi qu'à des acides dimandéliques, résultant d'une réaction subséquente, à savoir une deuxième condensation d'acide glyoxylique sur un acide mandélique. En outre, l'acide glyoxylique peut se transformer selon la réaction de Cannizzaro en acide oxalique et en acide glycolique.

Dans le but d'obtenir en particulier une sélectivité améliorée, la demande internationale de brevet WO 2009/077383 propose l'utilisation d'un réacteur à écoulement piston, avec ou sans garnissage. Cette solution peut cependant présenter certains inconvénients. D'une part, le déroulement d'une réaction dans un réacteur à écoulement piston est généralement peu flexible : il est difficile de faire varier les débits d'alimentation du réacteur dans une large mesure, ce qui implique que la production ne peut pas toujours être aisément adaptée aux besoins. D'autre part, les réacteurs munis de garnissage peuvent s'encrasser.

C'est pourquoi on pourra préférer dans certains cas conduire l'étape de condensation dans des réacteurs munis d'une agitation. Toutefois, on souhaite maintenir une bonne sélectivité de la réaction. De préférence, on souhaite atteindre au moins un de ces objectifs:

- un taux de transformation de l'acide glyoxylique élevé,
- un taux de transformation du composé aromatique hydroxylé réactif élevé,
- une sélectivité élevée en composé p-hydroxymandélique formé, par rapport au composé aromatique hydroxylé transformé et/ou par rapport à l'acide glyoxylique transformé,
- une sélectivité faible en composé secondaire ortho-hydroxymandélique formé, par rapport au composé aromatique hydroxylé transformé et/ou par rapport à l'acide glyoxylique transformé,
- une sélectivité faible en composé parasite di-hydroxymandélique formé, par rapport au composé aromatique hydroxylé transformé et/ou par rapport à l'acide glyoxylique transformé.

De plus, on ne souhaite pas que ce procédé ait un coût de fonctionnement trop élevé.

C'est dans ce contexte que les inventeurs ont cherché un procédé de préparation de composés p-hydroxymandéliques permettant de surmonter un ou plusieurs des inconvénients du réacteur à écoulement piston évoqués ci-dessus.

BREVE DESCRIPTION DE L'INVENTION

L'invention a pour objet un procédé de préparation d'un composé aromatique porteur d'au moins un groupe -CHOH-COOH en position para d'un groupe hydroxyle, comprenant au moins une étape de condensation d'au moins un composé aromatique porteur d'au moins un groupe hydroxyle et dont la position en para est libre, avec l'acide glyoxylique, la réaction de condensation étant conduite dans au moins un réacteur muni d'au moins un moyen de mélange, la puissance spécifique de mélange étant comprise entre 0,1 $kW/m^3$ et 15 $kW/m^3$.

En outre, l'invention a aussi pour objet un procédé de préparation d'un composé aromatique porteur d'au moins un groupe aldéhyde -CHO en position para d'un groupe hydroxyle, ce procédé comprenant les étapes consistant à :

- préparer un composé aromatique porteur d'au moins un groupe -CHOH-COOH en position para d'un groupe hydroxyle, selon le procédé décrit ci-dessus, puis
- oxyder ce composé.

BREVE DESCRIPTION DES FIGURES

La figure 1 représente schématiquement un mode de réalisation de l'invention dans lequel la réaction de condensation est conduite dans un réacteur agité.

La figure 2 représente schématiquement un autre mode de réalisation de l'invention dans lequel la réaction de condensation est conduite dans un réacteur muni d'une boucle de recirculation externe.

La figure 3 représente schématiquement un autre mode de réalisation de l'invention dans lequel la réaction de condensation est conduite dans une cascade de réacteurs agités.

La figure 4 représente schématiquement un autre mode de réalisation de l'invention dans lequel la réaction de condensation est conduite en parallèle dans plusieurs réacteurs agités.

DESCRIPTION DE L'INVENTION

Dans l'exposé qui suit, l'expression « compris entre ... et ... » doit être comprise comme incluant les bornes citées.

L'objet de la présente invention est un procédé permettant de préparer un composé aromatique porteur d'au moins un groupe -CHOH-COOH en position para d'un groupe hydroxyle. Pour cela, on effectue une réaction de condensation entre :

- d'une part au moins un composé aromatique porteur d'au moins un groupe hydroxyle et dont la position en para est libre,
- et d'autre part l'acide glyoxylique.

Dans l'exposé qui suit, l'expression « composé aromatique » désigne notamment un composé cyclique présentant des doubles liaisons délocalisées tel que défini dans la littérature, notamment par M. SMITH et J. MARCH Advanced Organic Chemistry, 5ème Edition, John Wiley and Sons, 1992, pp.46 et suivantes.

Le composé aromatique réactif peut être le phénol, mais aussi un phénol substitué ayant au moins une position en para du groupe hydroxyle non substituée. Le noyau aromatique du composé aromatique réactif est porteur d'au moins un groupe hydroxyle mais il peut être également porteur d'un ou plusieurs autres substituants. Généralement, par plusieurs substituants, on définit moins de quatre substituants par noyau aromatique. N'importe quel substituant peut être présent dans la mesure où il n'interfère pas dans la réaction de l'invention.

Ainsi, dans le procédé de l'invention, le composé aromatique porteur d'au moins un groupe hydroxyle et dont la position en para est libre peut être un composé de formule (I) :

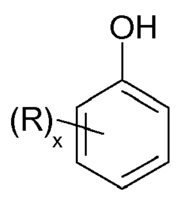
(I)

dans laquelle :

- au moins la position en para du groupe hydroxyle est libre,
- R représente un atome d'hydrogène ou un ou plusieurs substituants, identiques ou différents,
- x est un nombre inférieur ou égal à 4,
- lorsque x est supérieur à 1, deux groupes R placés sur deux atomes de carbone vicinaux peuvent former ensemble et avec les atomes de carbone qui les portent un cycle saturé, insaturé ou aromatique, ayant de 5 à 7 atomes et comprenant éventuellement un ou plusieurs hétéroatomes.

Dans la formule (I), les groupes R, identiques ou différents, peuvent représenter un atome d'hydrogène, un

groupe alkyle, alcényle, alkoxy, hydroxyalkyle, alkoxyalkyle, cycloalkyle, aryle, arylalkyle, un groupe hydroxyle, un groupe nitro, un atome d'halogène, un groupe halogéno ou perhalogénoalkyle, un groupe formyle, un groupe acyle ayant de 2 à 6 atomes de carbone, un groupe carboxylique, un groupe amino ou amido substitué ou non par un ou deux groupes alkyle ou phényle. Il est à noter que le groupe carboxylique peut être salifié, de préférence par un métal alcalin (sodium, potassium), ou estérifié par exemple par un groupe alkyle ou phényle.

Dans la formule (I), lorsque x est supérieur à 1, deux groupes R placés sur deux atomes de carbone vicinaux, peuvent être liés entre eux par un groupe alkylène, alcénylène ou alcénylidène ayant de 3 à 5 atomes de carbone pour former un cycle saturé, insaturé ou aromatique ayant de 5 à 7 atomes : un ou plusieurs (de préférence 2 ou 3) atomes de carbone pouvant être remplacés par un hétéroatome, de préférence l'oxygène.

Dans le cadre de l'invention, on entend par « alkyle », une chaîne hydrocarbonée linéaire ou ramifiée ayant de 1 à 15 atomes de carbone et de préférence de 1 ou 2 à 10 atomes de carbone. Des exemples préférés de groupes alkyles sont les groupes méthyle, éthyle, n-propyle, isopropyle, n-butyle, isobutyle, sec-butyle, tert-butyle.

Par « alkoxy », on entend un groupe alkyl-O- dans lequel le terme alkyle a la signification donnée ci-dessus. Des exemples préférés de groupes alkoxy sont les groupes méthoxy et éthoxy.

Par « alcényle », on entend un groupe hydrocarboné, linéaire ou ramifié, ayant de 2 à 15 atomes de carbone, comprenant une ou plusieurs doubles liaisons, de préférence, 1 à 2 doubles liaisons.

Par « cycloalkyle », on entend un groupe hydrocarboné cyclique, comprenant de 3 à 8 atomes de carbone, de préférence, un groupe cyclopentyle ou cyclohexyle.

Par « aryle », on entend un groupe monocyclique ou polycyclique aromatique, de préférence, monocyclique ou bicyclique comprenant de 6 à 12 atomes de carbone, de préférence, phényle ou naphtyle.

Par « arylalkyle », on entend un groupe hydrocarboné, linéaire ou ramifié porteur d'un cycle aromatique monocyclique et comprenant de 7 à 12 atomes de carbone, de préférence, benzyle.

Par « halogéno » ou « perhalogénoalkyle », on entend l'un des groupes suivants : $-CX_3$, $-[CX_2]_p-CX_3$ ou $-C_pH_aF_b$ ; dans lesdits groupes, X représente un atome d'halogène, de préférence un atome de chlore ou de fluor, p représente un nombre allant de 1 à 10, b un nombre allant de3à21 et a+b=2p+1.

Dans le cas où x est supérieur à 1, deux groupes R placés sur deux atomes de carbone vicinaux peuvent être liés entre eux par un groupe alkylène, alcénylène ou alcénylidène pour former un cycle saturé, insaturé ou aromatique ayant de 5 à 7 atomes formant ainsi, avec le cycle du composé aromatique, un bicycle. Des exemples de squelettes bicycliques préférés sont les suivants :

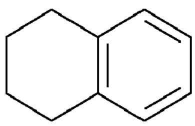

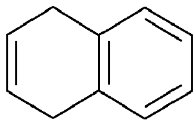

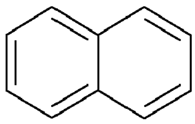

Dans le procédé de l'invention, le composé aromatique porteur d'au moins un groupe hydroxyle et dont la position en para peut avantageusement être choisi parmi les composés de formule (I) tels que décrit ci-dessus, dans laquelle R, identiques ou différents, représentent:

- un atome d'hydrogène,
- un groupe hydroxyle,
- un groupe alkyle, linéaire ou ramifié, ayant de 1 à 6 atomes de carbone, de préférence de 1 à 4 atomes de carbone, tel que méthyle, éthyle, n-propyle, isopropyle, n-butyle, isobutyle, sec-butyle, tert-butyle,
- un groupe alcényle, linéaire ou ramifié, ayant de 2 à 6 atomes de carbone, de préférence de 2 à 4 atomes de carbone, tel que vinyle, allyle,
- un groupe alkoxy linéaire ou ramifié ayant de 1 à 6 atomes de carbone, de préférence de 1 à 4 atomes de carbone tel que les groupes méthoxy, éthoxy, n-propoxy, isopropoxy, n-butoxy, isobutoxy, sec-butoxy, tert-butoxy,
- un groupe phényle,
- un atome d'halogène, de préférence un atome de fluor, chlore ou brome.

Dans la formule (I), le symbole « x » désigne le nombre de substituants sur le cycle, peut avantageusement être un nombre compris entre 0 et 4, de façon préférée égal à 0, 1 ou 2, et de façon encore plus préférée égal à 1.

De préférence, dans le procédé de l'invention, le composé aromatique porteur d'au moins un groupe hydroxyle et dont la position en para est libre, peut être choisi parmi les composés de formule (I) dans laquelle x est égal à 1 et R représente soit un atome d'hydrogène, soit un groupe alkyle ayant de 1 à 4 atomes de carbone.

A titre illustratif de composés répondant à la formule (I), on peut mentionner :

- ceux répondant à la formule (I) dans laquelle x est égal à 0, tels que :
    - le phénol,
- ceux répondant à la formule (I) dans laquelle x est égal à 1, tels que :
    - la pyrocatéchine
    - la résorcine
    - l'o-crésol
    - le m-crésol
    - le 2-éthylphénol
    - le 3-éthylphénol
    - le 2-propylphénol
    - le 2-sec-butylphénol
    - le 2-tert-butylphénol
    - le 3-tert-butylphénol
    - le 2-méthoxyphénol (également appelé gaïacol)
    - le 3-méthoxyphénol
    - le 2-éthoxyphénol (également appelé guétol)
    - le 2-isopropoxyphénol
    - l'aldéhyde salicylique
    - le salicylate de méthyle
    - le 2-chlorophénol
    - le 3-chlorophénol
    - le 3-nitrophénol
- ceux répondant à la formule (I) dans laquelle x est égal à 2, tels que :
    - le 2,3-diméthylphénol
    - le 2,5-diméthylphénol
    - le 3,5-diméthylphénol
    - le 2-hydroxy-5-acétamidobenzaldéhyde
    - le 2-hydroxy-5-éthamidobenzaldéhyde
    - le 2,3-dichlorophénol
    - le 2,5-dichlorophénol
    - le 3,5-dichlorophénol
    - le pyrogallol
- ceux répondant à la formule (I) dans laquelle x est égal à 3, tels que :
    - le 2,3,5-triméthylphénol
    - le 3,5-di-tertbutylphénol
    - le 2,3,5-trichlorophénol
- ceux répondant à la formule (I) présentant un groupe naphtalénique, tels que :
    - le 1-naphtol
    - le 2-naphtol
    - le 1,2-dihydroxynaphtalène
    - le 1,5-dihydroxynaphtalène
    - le 2,3-dihydroxynaphtalène
    - le 2,6-dihydroxynaphtalène
    - le 2,7-dihydroxynaphtalène
    - le 6-bromo-2-naphtol
- ceux répondant à la formule (I) présentant un enchaînement de noyaux benzéniques :
    - le 2-phénoxyphénol

- le 3-phénoxyphénol

De façon très préférée, dans le procédé de l'invention, le composé aromatique porteur d'au moins un groupe hydroxyle et dont la position en para est libre, peut être choisi dans le groupe constitué par le phénol, l'o-crésol, le m-crésol, le 3-éthylphénol, le 2-tert-butylphénol, le gaïacol et le guétol, et de façon encore plus préférée, il s'agit du gaïacol ou du guétol ou de leur mélange.
Selon un mode de réalisation préféré du procédé selon l'invention, un seul composé aromatique réactif peut être mis en oeuvre dans l'étape de condensation. Cependant, il n'est pas exclu que plusieurs composés aromatiques réactifs soient mis en oeuvre simultanément. Selon un autre mode de réalisation, un mélange de plusieurs composés aromatiques réactifs peut être mis en oeuvre, de préférence un mélange de deux composés aromatiques réactifs. De façon très préférée, il peut s'agir d'un mélange de gaïacol et de guétol.
Dans le procédé selon l'invention, le composé aromatique porteur d'au moins un groupe hydroxyle et dont la position en para est libre subit une réaction de condensation avec de l'acide glyoxylique. Le rapport molaire entre le composé aromatique hydroxylé et l'acide glyoxylique peut varier entre 1,0 et 4,0, de préférence entre 1,2 et 2,2.
Cette étape de condensation peut être conduite en milieu aqueux, en présence d'au moins un agent alcalin.
Dans le cas d'une mise en oeuvre en milieu aqueux, la concentration du composé aromatique hydroxylé réactif peut être comprise de préférence entre 0,5 et 1,5 moles/litre, et plus particulièrement aux environs de 1 mole/litre.
L'acide glyoxylique peut être utilisé en solution aqueuse ayant une concentration variant par exemple entre 15 % et 70 % en poids. On a recours, d'une manière préférée, aux solutions commerciales dont la concentration est d'environ 50 % en poids
L'agent alcalin conduit à la salification d'une part de la fonction alcool du composé aromatique hydroxylé, et d'autre part de la fonction carboxylique de l'acide glyoxylique puis du produit p-mandélique final. L'agent alcalin peut être un hydroxyde de métal alcalin, notamment l'hydroxyde de sodium ou l'hydroxyde de potassium. Pour des considérations économiques, l'hydroxyde de sodium peut être préféré. La solution d'hydroxyde de métal alcalin mise en oeuvre peut avoir une concentration comprise entre 10 % et 50 % en poids. La quantité d'hydroxyde de métal alcalin introduite dans le milieu réactionnel tient compte de la quantité nécessaire pour salifier la fonction hydroxyle du composé aromatique hydroxylé et la fonction carboxylique de l'acide glyoxylique. Généralement, la quantité d'hydroxyde de métal alcalin varie entre 80 % et 120 % de la quantité stoechiométrique.
Une variante possible consiste à effectuer la réaction en présence d'un catalyseur de type acide dicarboxylique, de préférence l'acide oxalique comme décrit dans la demande internationale de brevet WO 99/65853. La quantité de catalyseur mis en oeuvre, exprimée par le rapport entre le nombre de moles de catalyseur et le nombre de moles d'acide glyoxylique, peut être avantageusement choisie entre 0,5 % et 2,5 %, de préférence entre 1 % et 2 %.
Selon un mode de réalisation de la présente invention, le composé aromatique hydroxylé réactif et l'agent alcalin sont mélangés préalablement à la mise en contact du composé aromatique hydroxylé réactif avec l'acide glyoxylique. Ainsi, le procédé selon l'invention peut comprendre dans un premier temps la mise en contact du composé aromatique hydroxylé réactif avec un hydroxyde de métal alcalin en solution aqueuse, puis la mise en contact de la solution résultante avec l'acide glyoxylique. Ce mode de réalisation permet avantageusement de mieux maîtriser la température de la réaction car la réaction de salification de l'acide glyoxylique est exothermique.
Selon un autre mode de réalisation, le procédé selon l'invention comprend dans un premier temps la mise en contact de l'acide glyoxylique avec un hydroxyde de métal alcalin en solution aqueuse, puis la mise en contact de la solution résultante avec le composé aromatique hydroxylé réactif.
Selon encore un autre mode de réalisation, le procédé selon l'invention comprend d'une part la mise en contact du composé aromatique hydroxylé réactif avec l'agent alcalin en solution aqueuse, d'autre part la mise en contact de l'acide glyoxylique avec l'agent alcalin en solution aqueuse, puis la mise en contact des deux solutions résultantes.
Ces éventuelles étapes de mise en contact de l'acide glyoxylique avec un hydroxyde de métal alcalin en solution aqueuse et/ou de mise en contact du composé aromatique hydroxylé réactif avec l'agent alcalin peuvent être mises en oeuvre à une température comprise entre 10°C et 40°C, par exemple à 15°C ou à 35°C.
Le mélange réactionnel obtenu peut avoir une viscosité à 20°C comprise entre 0,5 mPa.s et 50 mPa.s, plus préférentiellement entre 1,5 mPa.s et 3 mPa.s. Selon l'invention, ce mélange est introduit dans au moins un réacteur, dans lequel la réaction de condensation a lieu.
Selon un premier mode de réalisation, la réaction de condensation est conduite au moins en partie dans un réacteur en régime discontinu, également appelé couramment régime « batch », ou en régime semi-continu, également appelé couramment régime « fed batch ». Dans un régime discontinu, il n'y a ni entrée ni sortie de matière durant la réaction. Dans un régime semi-continu, certains constituants sont ajoutés ou soustraits en cours de réaction. Par exemple, l'introduction d'un ou de plusieurs réactifs peut être effectuée pendant la réaction, mais il n'y a pas de sortie de matière, ou inversement. Pour pouvoir atteindre les débits désirés, il est possible d'opérer plusieurs réacteurs en parallèles. Les réacteurs peuvent être identiques ou différents. D'un point de vue industriel, la production en régime discontinu ou semi-continu peut ne pas être optimale. Pour remédier à cette difficulté, plusieurs réacteurs peuvent être opérés en parallèle

et leurs mises en oeuvre peuvent être décalées dans le temps de façon à avoir une production de produit plus continue dans le temps.

Selon un second mode de réalisation, la réaction de condensation est conduite au moins en partie dans un réacteur en régime continu. La réaction est conduite de façon très préférée dans une cascade de plusieurs réacteurs, de préférence d'au moins deux réacteurs, de façon plus préférée d'au moins trois réacteurs. Le nombre de réacteurs en cascade peut être compris entre 3 et 20, de façon plus préférée entre 4 et 10, et de façon encore plus préférée entre 5 et 8. Lorsqu'une cascade de réacteurs est mise en oeuvre, des réacteurs peuvent être identiques ou différents entre eux. Il peut s'agir d'une cascade de réacteurs agités, mais pas exclusivement. En outre, le dernier réacteur de la cascade de réacteurs peut être un réacteur finisseur, de volume différent des autres réacteurs, par exemple de volume plus important. Ce réacteur finisseur permet avantageusement d'ajuster selon les besoins les caractéristiques en sortie du procédé, par exemple les taux de rendement et de sélectivité du procédé.

Les conditions opératoires de la réaction peuvent être fixées en fonction des réactifs et du type de réacteur ou d'enchainement de réacteur mis en oeuvre.

La température de la réaction peut être comprise entre 10°C et 90°C. Selon un mode de réalisation, la température de la réaction peut être comprise entre 10°C et 20°C. Selon un autre mode de réalisation, la température peut être comprise entre 30°C et 40°C. De plus, la température peut varier au cours de la réaction. Par exemple, la réaction peut être conduite à une température comprise entre 10°C et 20°C pendant une certaine durée, puis la température peut être élevée entre 30°C et 50°C pendant une phase de finition.

La réaction peut être conduite à pression atmosphérique mais sous atmosphère contrôlée de gaz inertes, de préférence d'azote ou éventuellement de gaz rares, en particulier l'argon. On choisit préférentiellement l'azote.

Le temps de séjour total des réactifs en régime continu et le temps de cycle ou d'opération en régime discontinu peut varier grandement, par exemple de quelques minutes à plusieurs heures, voire plusieurs jours, notamment en fonction des conditions opératoires, en particulier en fonction de la température de la réaction. Lorsque la température est comprise entre 10°C et 20°C, le temps de séjour total des réactifs peut être compris entre 10 heures et 100 heures. Lorsque la température est comprise entre 30°C et 50°C, le temps de séjour total des réactifs peut être compris entre 30 minutes et 30 heures.

Dans le procédé selon l'invention, la réaction de condensation est conduite dans au moins un réacteur muni d'au moins un moyen de mélange. Ce moyen de mélange peut être choisi parmi les différents moyens connus de l'homme du métier.

Les inventeurs ont découvert que le mélange du fluide à l'intérieur du réacteur était un paramètre important pour le procédé de production selon l'invention. Pour obtenir un bon rendement et une bonne sélectivité lors de la réaction de condensation d'au moins un composé aromatique porteur d'au moins un groupe hydroxyle et dont la position en para est libre, avec l'acide glyoxylique, les inventeurs ont déterminé que la réaction devait être conduite dans au moins un réacteur muni d'au moins un moyen de mélange, auquel est appliqué une puissance spécifique de mélange comprise entre 0,1 $kW/m^3$ et 15 $kW/m^3$. De façon préférée, la puissance spécifique de mélange est comprise entre 0,1 $kW/m^3$ et 12 $kW/m^3$, de façon plus préférée entre 0,1 $kW/m^3$ et 10 $kW/m^3$, et de façon encore plus préférée entre 0,1 $kW/m^3$ et 5 $kW/m^3$.

Il a été constaté que, lorsqu'on diminue cette puissance à une valeur inférieure à une valeur seuil, le rendement de la réaction de condensation chute significativement. Les inventeurs ont découvert aussi l'existence d'une valeur plancher supérieure : l'augmentation de la puissance spécifique de mélange au-delà de ce seuil provoque alors une surconsommation énergétique du procédé, sans bénéfice sur le rendement de la réaction.

De façon générale, la puissance spécifique de mélange, exprimée en kilowatt par mètre cube de fluide à mélanger, peut être définie comme la puissance dissipée par le fluide, rapportée au volume de fluide mélangé. La puissance dissipée par le fluide peut être estimée à partir de la puissance consommée par les systèmes d'alimentation du ou des moyens de mélange, typiquement des moteurs et/ou des pompes. Par exemple, la puissance dissipée représente typiquement 80% de la puissance électrique consommée par le moteur ou la pompe à l'échelle industrielle.

Plusieurs modes de réalisation de la présente invention sont possibles, en fonction du moyen de mélange utilisé. Dans la présente invention, le réacteur de condensation peut être muni d'un ou de plusieurs moyens de mélange, ces moyens pouvant être identiques ou différents.

Selon un premier mode de réalisation, le moyen de mélange est un mobile d'agitation. La réaction est alors conduite dans un réacteur agité. Différents technologies de mobiles d'agitation sont connues de l'homme du métier et sont disponibles commercialement. Le mobile d'agitation peut notamment être choisi dans le groupe constitué par les mobiles radiaux, les mobiles axiaux, les mobiles mixtes, les mobiles doubles flux, les mobiles de brassage mécanique et les mobiles adaptés aux milieux visqueux ou chargés en solides.

Parmi les mobiles radiaux, on peut par exemple citer les turbines à pales droites, dites de Rushton, les turbines à pales incurvées, les turbines à pales courbes et les mobiles Phasejet de la société Ekato.

Parmi les mobiles axiaux, on peut par exemple citer les hélices marines, les hélices à grande pales minces, les mobiles A310 et A320 de la société Lightnin, les mobiles TTP et TT de la société Mixel, les mobiles Isojet et Viscoprop-F® de la société Ekato, les mobiles XE-3, HE-3 et SC-3 de la société Chemineer, et les mobiles LA, LB et LC de la société Lumpp.

Parmi les mobiles mixtes, on peut par exemple citer les turbines à 2, 4 ou 6 pales inclinées.

Parmi les mobiles doubles flux, on peut citer les mobiles Intermig® de la société Ekato.

Parmi les mobiles de brassage mécanique, on peut par exemple citer les ancres, les cadres, les impellers, et le mobile Maxblend de la société Sumimoto.

Parmi les mobiles adaptés aux milieux visqueux ou chargés en solides, on peut par exemple citer le double ruban hélicoïdal et les mobiles Paravisc et Koaxial de la société Ekato.

Le design et la taille du mobile d'agitation peuvent être choisis par l'homme du métier en fonction des performances de mélange désirées. La taille du mobile peut notamment être choisie pour que le rapport D (diamètre du mobile) / T (diamètre du réacteur) soit compris entre 0,3 et 0,7 dans le cas des mobiles radiaux, axiaux ou mixtes. Dans le cas des mobiles de brassage mécanique, le rapport D/T peut plus préférentiellement être compris entre 0,9 et 1.

Selon ce mode de réalisation, la puissance spécifique de mélange est égale à la puissance spécifique délivrée par le mobile d'agitation. Celle-ci peut être calculée en connaissant les données techniques fournies par le fabricant du mobile d'agitation, la vitesse de rotation du mobile, le diamètre du mobile, la masse volumique du fluide à mélanger et le volume du liquide à mélanger. Les formules suivantes peuvent notamment être appliquées :

$$\epsilon = \frac{P}{V} = \frac{N_p \times \rho \times N^3 \times D^5}{V}$$

dans laquelle

- ε est la puissance spécifique, en Watt/$m^3$,
- P est la puissance, en Watt
- V est le volume du liquide à mélanger, en $m^3$,
- Np est le nombre de puissance, c'est-à-dire une valeur adimensionnelle et tabulée donnée par le constructeur en fonction du mobile et des conditions hydrodynamiques,
- p est la masse volumique du fluide à mélanger, en kg/$m^3$,
- N est la vitesse d'agitation, en Hz ou tr/min, et
- D est le diamètre du mobile, en m.

Lorsque plusieurs mobiles sont disposés sur le même arbre d'agitation, leurs puissances spécifiques s'ajoutent.

La puissance spécifique massique d'agitation, en Watt/kg, correspond à la puissance spécifique (en Watt/$m^3$) divisée par la masse volumique du fluide.

En pratique, la puissance spécifique d'agitation, délivrée par un mobile, peut être calculée à partir de la puissance d'agitation P en Watt, c'est-à-dire la puissance dissipée dans le fluide mélangé, divisée par le volume de fluide contenu dans le réacteur. La puissance d'agitation P peut être estimée à partir de la puissance consommée par le moteur qui met en mouvement le mobile, par exemple la puissance électrique consommée par le moteur. P représente typiquement 80% de la puissance consommée par le moteur à l'échelle industrielle.

Selon ce premier mode de réalisation, la réaction de condensation est conduite dans au moins un réacteur agité muni d'un mobile d'agitation, la puissance spécifique d'agitation étant comprise entre 0,1 kW/$m^3$ et 15 kW/$m^3$. De façon plus préférée, la puissance spécifique d'agitation est inférieure à 10 kW/$m^3$, et de façon encore plus préférée, la puissance spécifique d'agitation est inférieure à 5 kW/$m^3$. Pour les mobiles d'agitation standard, la puissance spécifique d'agitation est de façon plus préférée comprise entre 0,5 kW/$m^3$ et 10 kW/$m^3$, et de façon encore plus préférée comprise entre 0,3 kW/$m^3$ et 5 kW/$m^3$. Pour les mobiles d'agitation à faible consommation d'énergie, connus sous le nom d'hydrofoils, la puissance spécifique d'agitation est de façon plus préférée comprise entre 0,1 kW/$m^3$ et 5 kW/$m^3$, et de façon encore plus préférée comprise entre 0,1 kW/$m^3$ et 2 kW/$m^3$. Les mobiles axiaux A310 et A320 de la société Lightnin, les mobiles axiaux TTP et TT de la société Mixel, les mobiles axiaux Isojet et Viscoprop-F® de la société Ekato et les mobiles axiaux XE-3, HE-3 et SC-3 de la société Chemineer font partie des hydrofoils.

En alternative aux mobiles d'agitation, le mélange du milieu réactif peut être assuré par d'autres moyens de mélanges, pouvant être considérés comme équivalents aux mobiles d'agitation.

Selon un second mode de réalisation de la présente invention, le moyen de mélange est un circuit de recirculation externe. Le réacteur dans lequel la réaction de condensation est conduite est dans ce cas équipé d'au moins un circuit externe de recirculation muni d'une pompe. Sous l'action de cette pompe, une partie du fluide contenu dans le réacteur est prélevée, circule dans un conduit externe et est réinjecté dans le réacteur. Cette mise en mouvement permet d'assurer le mélange de cette partie du fluide, et plus généralement de l'ensemble du fluide contenu dans le réacteur. Il est possible

de disposer sur le conduit externe de recirculation un échangeur de chaleur, de façon à maintenir le fluide à une température désirée. En outre ou alternativement, un autre dispositif de mélange, par exemple un rotor-stator, peut être disposé sur le conduit externe de recirculation. Selon ce mode de réalisation, le réacteur peut être opéré en régime discontinu, semi-continu ou continu.

Selon un troisième mode de réalisation, le moyen de mélange du réacteur selon l'invention est un dispositif oscillant. La société DRM propose notamment des mobiles oscillants sous la marque Fundamix®. L'oscillation du mobile dans le réacteur assure le mélange du milieu réactionnel. Un autre type de dispositif oscillant consiste en une colonne pulsée, par exemple un réacteur COBR (Continuous Oscillatory Baffled Reactor, selon la terminologie anglo-saxonne). De tels réacteurs sont par exemple commercialisés par la société Nitech. Selon ce mode de réalisation, le réacteur peut être opéré en régime discontinu, semi-continu ou continu. Lorsqu'il s'agit d'un réacteur de type colonne pulsé, on pourra opérer en régime discontinu grâce à une boucle de recyclage entre l'entrée et la sortie du réacteur.

Selon un quatrième mode de réalisation, le réacteur muni d'un moyen de mélange selon l'invention est un mélangeur planétaire. Un mélangeur planétaire consiste en un réacteur muni à la fois d'un disperseur et d'un mobile à recirculation globale. Selon ce mode de réalisation, le réacteur peut être opéré en régime discontinu, semi-continu ou continu.

Dans les trois cas ci-dessus, la puissance déployée par le moyen de mélange n'est pas dissipée par l'intégralité du fluide, mais un certain volume de ce fluide. Par exemple, dans le cas où le moyen de mélange est un circuit de recirculation externe, seul le fluide contenu dans le volume du corps de pompe reçoit la puissance délivrée par la pompe. Toutefois, au sens de la présente invention, la puissance spécifique de mélange de ces modes de réalisation est définie comme la puissance produite par le moyen de mélange dissipée par le fluide divisée par le volume total de fluide. Ainsi, dans le cas où le moyen de mélange est un circuit de recirculation externe, la puissance spécifique de mélange au sens de l'invention est la puissance produite par la pompe de recirculation dissipée par le fluide divisée par le volume total de fluide dans le réacteur. Dans le cas où le moyen de mélange est un mobile oscillant, la puissance spécifique de mélange au sens de l'invention est la puissance dissipée par le fluide produite par le moteur du mobile divisée par le volume total de fluide dans le réacteur. Dans le cas où le moyen de mélange est un COBR, la puissance spécifique de mélange au sens de l'invention est la puissance totale dissipée par le fluide produite par la pompe mettant en mouvement le fluide dans le réacteur et par le dispositif générant les pulsations divisée par le volume total de fluide dans le réacteur. Enfin, dans le cas où la réaction est conduite dans un mélangeur planétaire, la puissance spécifique de mélange au sens de l'invention est la puissance dissipée par le fluide produite par le moteur du disperseur et du mobile à recirculation divisée par le volume total de fluide dans le réacteur.

Enfin, selon un cinquième mode de réalisation, le moyen de mélange consiste en une structuration du réacteur. La réaction de condensation est alors conduite dans un réacteur structuré. La structuration peut être choisie parmi les mélangeurs statiques 2D ou 3D, les mousses métalliques ou céramiques, les garnissages vrac et les structurations géométriques. Des sociétés telles que Sulzer, Kenics-Chemineer et Verder proposent de nombreux types de structuration. Par ailleurs, la réaction peut être conduite dans un tube droit fin. Selon ce mode de réalisation, la puissance spécifique de mélange au sens de l'invention est la puissance dissipée par le fluide produite par la pompe mettant en mouvement le fluide dans le réacteur divisée par le volume total de fluide dans le réacteur. Cette puissance spécifique peut ici être déduite en calculant les pertes de charges subies par le fluide dans le réacteur structuré. De préférence, dans ce mode de réalisation, le réacteur est opéré en régime discontinu, rendu possible grâce à une boucle de recyclage entre l'entrée et la sortie du réacteur.

En fin de réaction, on obtient un composé aromatique porteur d'au moins un groupe -CHOH-COOH en position para d'un groupe hydroxyle. Ce composé peut être choisi parmi les composés représentés par la formule (II) suivante :

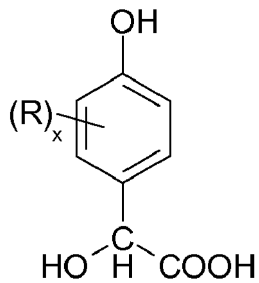

(II)

dans laquelle R et x ont les significations données dans la formule (I).

De préférence, le composé aromatique porteur d'au moins un groupe -CHOH-COOH en position para d'un groupe hydroxyle est choisi parmi l'acide 4-hydroxy-3-méthoxy-mandélique, l'acide 4-hydroxy-3-éthoxy-mandélique, et un mélange de ceux-ci.

Lorsque la réaction est conduite en milieu aqueux en présence d'un agent alcalin, le composé p-hydroxymandélique obtenu peut être sous forme salifiée. Une étape de neutralisation peut alors être effectuée pour obtenir le composé p-hydroxymandélique portant une fonction acide tel que décrit ci-dessus.

Ces produits sont particulièrement intéressants car ce sont des produits intermédiaires permettant entre autres, d'obtenir par réduction, des acides hydroxyarylacétiques ou par oxydation, des acides hydroxyarylglyoxyliques (i.e. hydroxyaryl $\alpha$-oxoacétiques) ou des aldéhydes hydroxyaromatiques.

Une application préférée de l'invention est la préparation d'aldéhydes hydroxyaromatiques, par oxydation des composés de formule (II) obtenus selon l'invention.

A l'issue de la réaction de condensation, on peut séparer le composé p-hydroxymandélique obtenu du mélange réactionnel selon les techniques classiques de séparation, notamment par cristallisation ou par extraction à l'aide d'un solvant organique approprié. Une étape de neutralisation peut être effectuée.

Alternativement, le mélange réactionnel obtenu à l'issue de la réaction de condensation peut être utilisé tel quel. Toutefois, il est préférable de récupérer le composé aromatique hydroxylé qui n'a pas réagi.

A cet effet, on peut mettre en oeuvre les traitements décrits dans l'état de la technique, notamment le traitement décrit dans le brevet FR 2379501. Il consiste à ajouter un acide minéral, par exemple l'acide chlorhydrique ou sulfurique, pour ajuster le pH entre 5 et 7, puis à extraire le composé aromatique hydroxylé qui n'a pas réagi dans un solvant organique, notamment dans l'éther ou le toluène. Après extraction, les phases aqueuse et organique peuvent être séparées.

La présente invention a également pour objet un procédé de préparation d'un composé aromatique porteur d'au moins un groupe aldéhyde -CHO en position para d'un groupe hydroxyle, ce procédé comprenant les étapes consistant à :

- préparer un composé aromatique porteur d'au moins un groupe -CHOH-COOH en position para d'un groupe hydroxyle, selon le procédé décrit ci-avant, puis
- oxyder ce composé.

L'oxydation peut être conduite selon les techniques décrites dans la littérature. Ainsi, on peut se référer à P. HEBERT (Bull. Soc. Chim. France, 27, p.45-55, 1920) et à NAGAI SHIGEKI et al, (JP-A 76/128934). L'oxydation est généralement conduite en atmosphère oxydante comme l'oxygène ou l'air, en milieu basique et en présence d'un catalyseur approprié.

Ainsi, l'invention permet d'accéder facilement au 4-hydroxybenzaldéhyde et à la vanilline et ses analogues, par exemple 3-éthyl-vanilline, 3-isopropylvanilline, par oxydation respectivement de l'acide p-hydroxymandélique et des acides 4-hydroxy-3-méthoxymandélique, 3-éthoxy-4-hydroxy-mandélique et 4-hydroxy-3-isopropoxymandélique.

EXEMPLES

La figure 1 représente schématiquement un mode de réalisation de l'invention dans lequel la réaction de condensation est conduite dans un réacteur agité. Le réacteur 1 est muni d'un moyen d'agitation qui consiste en un mobile d'agitation 2. Ce mobile est entrainé par un moteur 3. Le mobile d'agitation 2 permet de mélanger le milieu réactionnel 6. Sur la figure 1, le réacteur 1 est opéré en régime continu : les réactifs sont introduits par l'entrée 4 et sont soutirés par la sortie 5. Toutefois, l'invention n'est pas limitée à ce mode de réalisation, et ce réacteur agité peut également être mis en oeuvre en régime discontinu ou semi-continu.

La figure 2 représente schématiquement un autre mode de réalisation de l'invention dans lequel la réaction de condensation est conduite dans un réacteur 7 muni d'un circuit de recirculation externe 8. Le fluide est mis en mouvement dans le circuit de recirculation externe 8 grâce à la pompe 9. Le circuit de recirculation externe 8 assure le mélange du milieu réactionnel 10.

La figure 3 représente schématiquement un autre mode de réalisation de l'invention dans lequel la réaction de condensation est conduite dans une cascade de réacteurs agités 11. Chaque réacteur 11 est muni d'un moyen de mélange, ici un mobile d'agitation 12. Sur cette figure 3, trois réacteurs agités 11 sont représentés. Toutefois, ce nombre n'est pas limité à trois. En sortie du dernier réacteur agité 11, le milieu réactionnel est introduit dans un réacteur finisseur 13. Sur la figure 3, le réacteur finisseur 13 est un réacteur tubulaire, à écoulement de type piston. Toutefois, l'invention n'est pas limitée à ce mode de réalisation, et le réacteur finisseur peut par exemple être un réacteur agité.

La figure 4 représente schématiquement un autre mode de réalisation de l'invention dans lequel la réaction de condensation est conduite en parallèle dans plusieurs réacteurs agités. Les réacteurs agités 14, 15, 16 et 17 sont disposés en parallèles et sont opérés en régime discontinus ou semi-continu, chacun étant respectivement alimenté

en réactifs par les conduits 18, 19, 20 et 21. Grace à l'opération en parallèle des quatre réacteurs, le flux de produits obtenu en 22 peut être lissé.

## Revendications

1. Procédé de préparation d'un composé aromatique porteur d'au moins un groupe -CHOH-COOH en position para d'un groupe hydroxyle, comprenant au moins une étape de condensation d'au moins un composé aromatique porteur d'au moins un groupe hydroxyle et dont la position en para est libre, avec l'acide glyoxylique, la réaction de condensation étant conduite dans au moins un réacteur muni d'au moins un moyen de mélange, la puissance spécifique de mélange étant comprise entre 0,1 kW/$m^3$ et 15 kW/$m^3$.

2. Procédé selon la revendication 1, **caractérisé en ce que** le composé aromatique porteur d'au moins un groupe hydroxyle et dont la position en para est libre, est choisi dans le groupe constitué par le phénol, l'o-crésol, le m-crésol, le 3-éthylphénol, le 2-tert-butylphénol, le gaïacol et le guétol, et de façon encore plus préférée, il s'agit du gaïacol ou du guétol ou de leur mélange.

3. Procédé selon l'une ou l'autre des revendications 1 et 2, **caractérisé en ce que** la réaction de condensation est conduite dans un réacteur en régime discontinu ou en régime semi-continu.

4. Procédé selon la revendication 3, **caractérisé en ce que** plusieurs réacteurs sont opérés en parallèles.

5. Procédé selon l'une ou l'autre des revendications 1 et 2, **caractérisé en ce que** la réaction de condensation est conduite dans un réacteur en régime continu.

6. Procédé selon la revendication 5, **caractérisé en ce que** la réaction est conduite dans une cascade de plusieurs réacteurs, de préférence d'au moins deux réacteurs, de façon plus préférée d'au moins trois réacteurs.

7. Procédé selon la revendication 6, **caractérisé en ce que** le dernier réacteur de la cascade de réacteur est un réacteur finisseur.

8. Procédé selon l'une quelconque des revendications 1 à 7, **caractérisé en ce que** la puissance spécifique de mélange est comprise entre 0,1 kW/$m^3$ et 12 kW/$m^3$, de façon plus préférée entre 0,1 kW/$m^3$ et 10 kW/$m^3$, et de façon encore plus préférée entre 0,1 kW/$m^3$ et 5 kW/$m^3$.

9. Procédé selon l'une quelconque des revendications 1 à 8, **caractérisé en ce que** le moyen de mélange du réacteur est un mobile d'agitation, pouvant de préférence être choisi dans le groupe constitué par les mobiles radiaux, les mobiles axiaux, les mobiles mixtes, les mobiles de brassage mécanique et les mobiles adaptés aux milieux visqueux ou chargés en solides.

10. Procédé de préparation d'un composé aromatique porteur d'au moins un groupe aldéhyde -CHO en position para d'un groupe hydroxyle, ce procédé comprenant les étapes consistant à :

    - préparer un composé aromatique porteur d'au moins un groupe -CHOH-COOH en position para d'un groupe hydroxyle, selon le procédé décrit dans l'une quelconque des revendications 1 à 9, puis
    - oxyder ce composé.

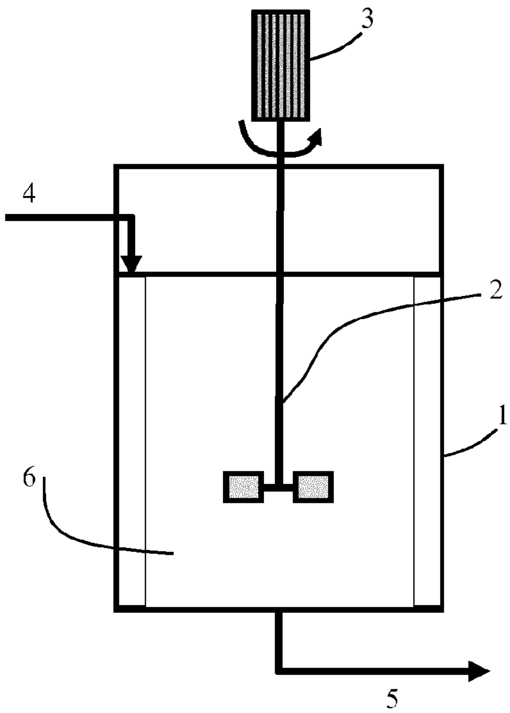


FIGURE 1

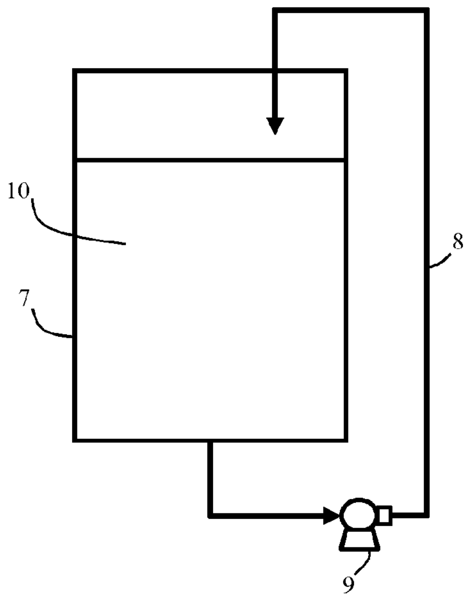


FIGURE 2

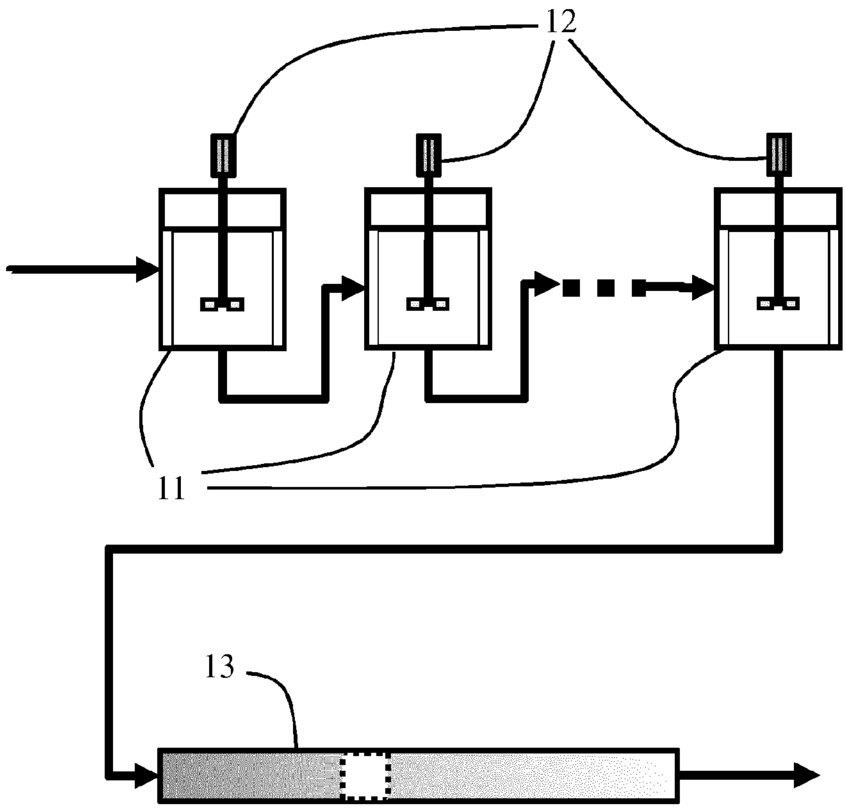


FIGURE 3

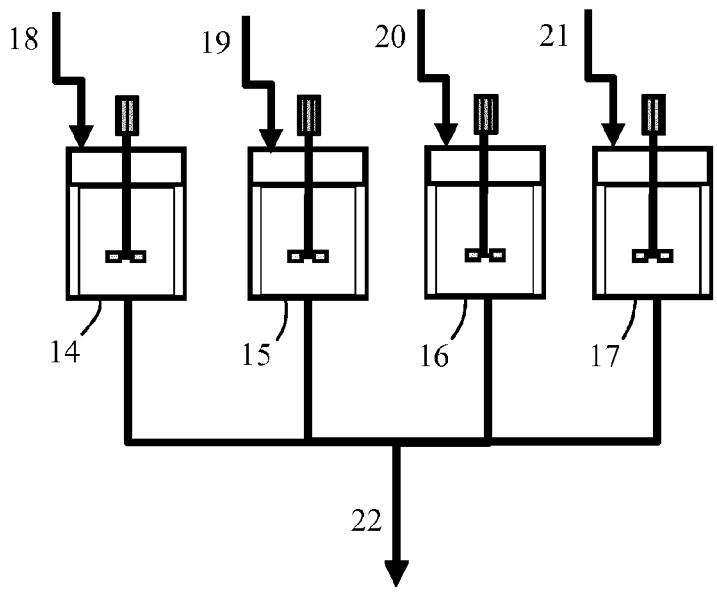


FIGURE 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2009077383 A
- WO 9965853 A
- FR 2379501
- JP 51128934 A, NAGAI SHIGEKI


### Littérature non-brevet citée dans la description


- KIRK-OTHMER - Encyclopedia of Chemical Technology. 1997, vol. 24, 812-825
- **M. SMITH ; J. MARCH.** Advanced Organic Chemistry. John Wiley and Sons, 1992, 46
- **P. HEBERT.** *Bull. Soc. Chim. France,* 1920, vol. 27, 45-55